(19) Europäisches Patentamt
European Patent Office
Office européen des brevets

(11) EP 3 963 321 B1

(12) # EUROPEAN PATENT SPECIFICATION

(45) Date of publication and mention of the grant of the patent:
**17.09.2025 Bulletin 2025/38**

(21) Application number: **20723847.8**

(22) Date of filing: **01.05.2020**

(51) International Patent Classification (IPC):
***G01N 33/50*** *(2006.01)* ***C12Q 1/6897*** *(2018.01)*
***C12N 5/0783*** *(2010.01)*

(52) Cooperative Patent Classification (CPC):
**G01N 33/5023; C12Q 1/6897; G01N 33/505;**
G01N 2500/04; G01N 2500/10

(86) International application number:
**PCT/EP2020/062206**

(87) International publication number:
**WO 2020/221924 (05.11.2020 Gazette 2020/45)**

(54) **ASSAYS**

TESTS

ANALYSES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.05.2019 GB 201906127**

(43) Date of publication of application:
**09.03.2022 Bulletin 2022/10**

(60) Divisional application:
**25194907.9**

(73) Proprietor: **Immutep S.A.S.**
**91190 Saint-Aubin (FR)**

(72) Inventors:
- **TRIEBEL, Frédéric**
  **91190 Saint-Aubin (FR)**
- **BRIGNONE, Chrystelle**
  **91190 Saint-Aubin (FR)**
- **ANGIN, Mathieu**
  **91190 Saint-Aubin (FR)**

(74) Representative: **Reddie & Grose LLP**
**The White Chapel Building**
**10 Whitechapel High Street**
**London E1 8QS (GB)**

(56) References cited:
**US-A1- 2017 097 333**

- **ANONYMOUS: "LAG3 / IL-2 Reporter - Jurkat Recombinant Cell Line", 31 December 2016 (2016-12-31), XP055710769, Retrieved from the Internet <URL:https://bpsbioscience.com/lag3-il-2-reporter-jurkat-recombinant-cell-line-79813> [retrieved on 20200701]**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 963 321 B1

## Description

This invention relates to assays for screening for, or determining activity of (including determining the potency of a preparation of), an agonist of lymphocyte-activation gene 3 (LAG-3). The invention also relates to kits for carrying out the assays.

Stimulation of T cell function is initiated upon interaction of the T cell receptor (TCR) with short peptides presented by MHC class I or II molecules (MHC I for CD8 T cells, MHC II for CD4 T cells) on the surface of antigen-presenting cells (APCs). in primary T cell, the TCR by itself is not capable of activating downstream pathways to initiate T cell activation. This also requires co-receptors, such as CD4 for helper T cells, and CD8 for cytotoxic T cells. These co-receptors bind their respective MHC molecules and stabilise the interaction of T cells and APCs. In addition to TCR binding to antigen-loaded MHC, both helper T cells and cytotoxic T cells require a number of secondary signals to become fully activated and respond. In the case of helper T cells, the first of these is provided by CD28. This protein is the receptor for two molecules expressed on the APC (CD80 and CD86) and initiates T-cell proliferation, leading to the expansion of T cell clones specific for the antigen. Cytotoxic T cells are less reliant on CD28 for activation, but do require signals from other co-stimulatory molecules such as CD70 and CD137.

The TCR is located in close proximity to a complex of signaling molecules, which mediate T cell activation through multiple signaling cascades (see Figure 1 for an overview of TCR signaling). These signaling molecules include the CD3 family of proteins. Once the TCR is properly engaged with the peptide-MHC complex, conformational changes in the associated CD3 chains are induced, which leads to their phosphorylation and association with downstream proteins. The TCR zeta chain is also phosphorylated upon TCR engagement. These molecules are phosphorylated by the Src kinases, leukocyte-specific tyrosine kinase (LCK) and Fyn, via their C-terminal Immunoreceptor Tyrosine-based Activation Motifs (ITAMs).

Phosphorylated CD3 ITAMs recruit and activate the Syk family kinase zeta-activated protein 70 kDa (ZAP70). ZAP70 then phosphorylates a membrane associated scaffolding protein called Linker for Activation of T cells (LAT). LAT in turn recruits a second molecular scaffold, SH2-domain-containing Leukocyte Protein of 76 kDa (Slp-76). Slp-76 is then phosphorylated by ZAP70 and the resulting LAT-Slp-76 complex acts as a scaffold for the recruitment of signalling effector molecules. Interleukin-2 inducible tyrosine kinase (ITK) then interacts with the LAT-Slp-76 complex and becomes activated by auto-phosphorylation. This promotes phosphorylation of the effector molecule phospholipase C gamma (PLC-$\gamma$1). PLC-$\gamma$1 transduces TCR signals by cleaving phosphatidylinositol triphosphate ($PIP_2$) in the plasma membrane to generate the second messengers diacylglycerol (DAG) and inositol trisphosphate ($IP_3$).

DAG, a membrane associated lipid, activates a number of downstream proteins, including various isoforms of protein kinase C (PKC) and RAS guanyl nucleotide-releasing protein (RasGRP). Upon activation by DAG, PKC-theta participates in activating the NF-$\kappa$B pathway, whereas RasGRP is a crucial activator of MAPK signalling pathways. $IP_3$ stimulates the efflux of $Ca^{2+}$ from the endoplasmic reticulum to the cytoplasm. Elevated $Ca^{2+}$ levels induce activation of the protein phosphatase, calcineurin, which then dephosphorylates the T cell transcription factor nuclear factor of activated T cells (NFAT). Dephosphorylated NFAT then migrates to the nucleus to join other transcription factors in inducing the transcription of specific genes.

Uncontrolled immune responses to pathogens or self-antigens can cause inflammatory tissue damage and autoimmune diseases. To prevent this, immune responses are regulated by a balance between co-stimulatory and inhibitory signals, collectively referred to as immune checkpoints, which are necessary for maintaining self-tolerance and protecting the host from tissue damage. Activated T cells express multiple co-inhibitory receptors, such as lymphocyte-activation gene 3 (LAG-3), programmed cell death protein 1 (PD-1), cytotoxic T-lymphocyte-associated protein 4 (CTLA-4), and T cell immunoglobulin and immunoreceptor tyrosine-based inhibitory motif [ITIM] domain (TIGIT). Inhibitory immune checkpoint receptors have been shown to modulate T cell responses to self proteins as well as to chronic infections and tumor antigens. Inhibitory immune checkpoint receptors are targets for cancer immunotherapy due to their potential for use in multiple types of cancers.

LAG-3 is a CD4 homolog type I membrane protein with four extracellular Ig superfamily domains. Similar to CD4, LAG-3 oligomerizes at the surfaces of T cells and binds to MHC class II molecules on APCs, but with significantly higher affinity than CD4. LAG-3 is expressed on activated CD4-positive and CD8-positive T lymphocytes where it associates with the CD3-TCR complex at the cell surface and negatively regulates signal transduction. As a consequence, it negatively regulates T cell proliferation, function, and homeostasis. When recognition of the MHC class II-peptide complex by a specific TCR occurs, intracellular signals are transduced in the T cell through the TCR and in the APC through MHC class II molecules. The negative regulatory role of LAG-3 signalling into T cells operates in primary $CD4^+$ and $CD8^+$ human T-cell responses (Maçon-Lemaître, et al., Immunology. 2005 Jun; 115(2):170-178). However, the molecular mechanism by which LAG-3 negatively regulates signal transduction in T cells is not known. The inhibitory function of LAG-3 requires its intracellular (IC) region, but this region does not contain a typical signalling motif with a known signalling mechanism. Maeda et al. (J. Biol. Chem. 2019, RA119.007455) have recently reported that LAG-3 transduces two independent inhibitory signals through an FxxL motif in the membrane-proximal region and the C-terminal EX repeat. However, these motifs have not previously been reported for inhibitory co-receptors, and the molecular mechanism of the inhibitory signal transduced by LAG-3 remains elusive.

WO 2017/037203 describes antibodies (such as the humanized monoclonal antibody IMP761), and antigen-binding fragments thereof, that are agonists of LAG-3, and their use for the treatment of conditions associated with proliferation and/or activation of $CD4^+$ and/or $CD8^+$ T cells, in particular inflammatory and autoimmune disorders.

Conventional methods used to measure the activity of antibody and other drugs designed to target immune checkpoint receptors rely on primary human cells and measurement of functional endpoints, such as cell proliferation, cell surface marker expression, and cytokine production. These assays are laborious and highly variable due to their reliance on donor primary cells, complex assay protocols, and unqualified assay reagents. As a result, these assays are difficult to establish in quality-controlled drug development settings. To address these difficulties, cell-based bioluminescent reporter bioassays have been developed by Promega Corporation for individual and combination immune checkpoint immunotherapy targets (Cheng et al., June 2016, Promega, "Quantitative Cell-Based Bioassays for Indivisual and Combination Immune Checkpoint Immunotherapy Targets*").*

The bioassays developed by Promega include Blockade Bioassays for PD-1, CTLA-4, LAG-3, and TIGIT. These assays rely on use of Jurkat T cells, which have been genetically engineered to express the co-inhibitory receptor of interest on the surface, and which contain a firefly luciferase reporter gene under the control of an NFAT response element (NFAT-RE). These cells express endogenous TCR, CD3 and CD28 receptors. When the cells are engaged with an appropriate ligand, the TCR transduces intracellular signals resulting in increased NFAT-RE-mediated luminescence. The bioluminescent signal is detected and quantified using a luciferase substrate and a standard luminometer. The assays also use artificial antigen-presenting cells (aAPCs) expressing an engineered cell surface protein designed to activate TCRs in an antigen-independent manner, and expressing a natural ligand for the co-inhibitory receptor on their surface (for the PD-1, CTLA-4 and TIGIT Blockade Bioassays), or Raji cells in the presence of a Staphylococcal Enterotoxin E (SEE) Superantigen (for the LAG-3 Blockade Bioassay - Raji cells naturally express MHC class II, a LAG-3 ligand). TCR engagement on the Jurkat cells induces luciferase activity. Co-engagement of the co-inhibitory receptor with its natural ligand inhibits luciferase activity.

Antibody-mediated blockade of binding of the co-inhibitory receptor to its natural ligand restores luciferase activity.

US 2017/097333 A1 describes a human LAG-3 functional assay using a Jurkat T-cell lymphoma cell line engineered to overexpress LAG-3 at an optimal level relative to CD3.

These bioassays demonstrate the performance required for use in antagonist antibody screening, potency testing, and stability studies. However, they are not suitable for testing agonist antibodies, such as LAG-3 agonist antibody (for example, IMP761, described in WO 2017/037203). In particular, the presence of natural ligand for LAG-3 in the blockade bioassay (such as MHC class II expressed Raji cells) would interfere with testing of an agonist. There is, therefore, a need to provide *in vitro* assays for identifying and testing agonists of LAG-3.

It has now surprisingly been found that IMP761, through agonism of LAG-3, inhibits TCR signaling, in particular NFAT-regulated gene expression, in LAG-3 positive T-cells. The applicant has appreciated that this can form the basis of *in vitro* bioassays to determine activity of a LAG-3 agonist, or a preparation of a LAG-3 agonist, and to identify new LAG-3 agonists.

According to the invention there is provided an *in vitro* assay for determining activity of an agonist of lymphocyte-activation gene 3 (LAG-3), which comprises:

providing a plurality of effector T cells, each effector T cell expressing LAG-3 and a T-cell receptor (TCR) on its surface, and comprising a reporter gene encoding a reporter, wherein expression of the reporter is regulated by LAG-3-mediated inhibition of TCR signaling within the effector T cells; and

determining the activity of the agonist from the extent to which expression of the reporter is altered (i.e. increased or decreased) in the presence of the agonist compared with expression of the reporter in the absence of the agonist;

wherein the assay is carried out in the absence of a natural ligand for LAG-3, wherein the natural ligand is MHC class II.

Assays of the invention for determining activity of the agonist include assays for determining the potency of a preparation of the agonist, for example, as part of a quality control step in production of the agonist (in particular, as a cell-based potency assay required for product release according to Good Manufacturing Practice, GMP), or for stability testing of a preparation of the agonist, for example, after a period of storage, or as a product characterization assay.

There is also provided according to the invention an *in vitro* assay for screening for an agonist of LAG-3, which comprises:

providing a plurality of effector T cells, each effector T cell expressing LAG-3 and a T-cell receptor (TCR) on its surface, and comprising a reporter gene encoding a reporter, wherein expression of the reporter is regulated by LAG-3-mediated inhibition of TCR signaling within the effector T cells; and

determining whether a candidate agonist is an agonist of LAG-3 by determining the extent to which expression of the reporter is altered (i.e. increased or decreased) in the presence of the candidate agonist compared with expression of the reporter in the absence of the candidate agonist;

wherein the assay is carried out in the absence of a natural ligand for LAG-3, wherein the natural ligand is MHC class II.

Assays of the invention for screening for an agonist of LAG-3 can be used to identify an agonist of LAG-3, for example, from a library of candidate agonists. Such candidate agonists may be drugs (e.g. synthetic small molecules), or biological agents, such as recombinant or natural proteins, antibodies, or fragments or derivatives thereof.

The reporter may be expressed in the effector T cells in the absence of the agonist, or candidate agonist.

Optionally expression of the reporter is reduced in the presence of the agonist, or candidate agonist, compared with expression of the reporter in the absence of the agonist, or candidate agonist.

For example, LAG-3-mediated inhibition of TCR signaling within the effector T cells may cause a reduction in a basal level of expression of the reporter in the effector T cells (see Figure 2(a)). Thus, activity of an agonist of LAG-3 (including the potency of a preparation of an agonist of LAG-3) may be determined, or an agonist of LAG-3 may be identified, by determining the extent to which the basal level of expression of the reporter is reduced in the presence of the agonist, or candidate agonist, compared with the basal level of expression of the reporter in the absence of the agonist, or candidate agonist.

The term "LAG-3-mediated inhibition of TCR signaling within the effector T cells" is used herein to mean that agonism of LAG-3 expressed on the surface of the effector T cells inhibits TCR-mediated signal transduction within the effector T cells, with a consequential alteration (i.e. an increase, or a decrease) in expression of the reporter. Any TCR-mediated signalling pathway within the effector T cells may be inhibited. For example, expression of the reporter gene may be under the control of a promoter or a response element (RE) which is responsive to binding of a transcription factor which is part of the signaling pathway. For example, the calcineurin/NFAT signaling pathway within the effector T cells may be inhibited. In particular, expression of the reporter gene may be under the control of an NFAT response element, such that a reduction in calcineurin/NFAT signaling, following agonism of LAG-3, causes a reduction in expression of the reporter from the reporter gene.

Expression of the reporter may be altered (i.e. increased or decreased) in each effector T cell in response to activation of the effector T cell via the TCR. Optionally assays of the invention further comprise: activating the effector T cells by antigen-independent, MHC class II-independent, TCR-mediated T-cell activation in the presence and absence of the agonist, or candidate agonist; and determining the activity (including the potency of a preparation) of the agonist, or the candidate agonist, from the extent to which expression of the reporter, in response to activation of the effector T cells, is altered in the presence of the agonist, or candidate agonist, compared with expression of the reporter, in response to activation of the effector T cells, in the absence of the agonist, or candidate agonist.

Thus, described herein is an *in vitro* assay for determining activity of an agonist of lymphocyte-activation gene 3 (LAG-3), which comprises:

providing a plurality of effector T cells, each effector T cell expressing LAG-3 and a T-cell receptor (TCR) on its surface, and comprising a reporter gene encoding a reporter, expression of which is altered in response to activation of the effector T cell via the TCR, wherein expression of the reporter is regulated by LAG-3-mediated inhibition of TCR signaling within the effector T cells;

activating the effector T cells by antigen-independent, MHC class II-independent, TCR-mediated T-cell activation in the presence and absence of the agonist; and

determining the activity of the agonist from the extent to which expression of the reporter, in response to activation of the effector T cells, is altered in the presence of the agonist compared with expression of the reporter, in response to activation of the effector T cells, in the absence of the agonist.

Described herein is also an *in vitro* assay for screening for an agonist of LAG-3, which comprises:

providing a plurality of effector T cells, each effector T cell expressing LAG-3 and a T-cell receptor (TCR) on its surface, and comprising a reporter gene encoding a reporter, expression of which is altered in response to activation of the effector T cell via the TCR, wherein expression of the reporter is regulated by LAG-3-mediated inhibitoin of TCR signaling within the effector T cells;

activating the effector T cells by antigen-independent, MHC class II-independent, TCR-mediated T-cell activation in the presence and absence of the candidate agonist; and

determining the activity of the candidate agonist from the extent to which expression of the reporter, in response to activation of the effector T cells, is altered in the presence of the candidate agonist compared with expression of the reporter, in response to activation of the effector T cells, in the absence of the candidate agonist.

Optionally expression of the reporter is increased in each effector T cell in response to activation of the effector T cell via the TCR, and is reduced in the presence of the agonist, or candidate agonist, as a result of LAG-3-mediated inhibition of TCR signaling within the effector T cell, and wherein the activity of the agonist, or candidate agonist, is determined from the extent to which expression of the reporter, in response to activation of the effector T cells, is reduced in the presence of the agonist, or candidate agonist, compared with expression of the reporter, in response to activation of the effector T cells, in the absence of the agonist, or candidate agonist.

The greater the extent to which expression of the reporter is altered in response to activation of the effector T cells in the presence of the agonist, or the candidate agonist, compared with expression of the reporter in response to activation of the effector T cells in the absence of the agonist, or the candidate agonist, the greater the activity (or potency of a preparation) of the agonist, or the candidate agonist, for LAG-3.

Optionally the effector T-cells are activated by cell-free T-cell activation.

The term "cell-free T-cell activation" is used herein to refer to activation of the effector T cells by use of one or more cell-free T-cell activators in the absence of any cells, other than the effector T cells. In particular, cell-free T-cell activation occurs without use of an antigen-presenting cell (APC), an artificial APC (aAPC), or any other MHC class I-, or MHC class II-expressing cell, such as a Raji cell.

Cell-free activation of T cells is advantageous because cell-based reagents require special storage conditions. Cells are typically kept in frozen storage and must then be thawed before use.

Reference to "activation of the effector T cell via the TCR" is used herein to mean that upon engagement of the TCR by a T-cell activator, a signal is transduced by the TCR within the effector T cell, resulting in an alteration (i.e. an increase, or a decrease) in expression of the reporter within the effector T cell. For example, expression from the reporter gene may be under the control of a promoter or a response element (RE) which is responsive to an activation signal of the effector T cells. For example, the response element may be bound by one or more transcription factors following activation of the effector T cell via the TCR.

Optionally the effector T cells are activated by contacting the effector T cells with an antigen-independent, MHC class II-independent, T-cell activator under conditions for antigen-independent, MHC class II-independent, TCR-mediated activation of the effector T cells by the T-cell activator.

There is also provided according to the invention a kit for carrying out an *in vitro* assay for determining activity of, or screening for, an agonist of LAG-3, which comprises:

a plurality of effector T cells, each effector T cell expressing LAG-3 and a T-cell receptor (TCR) on its surface, and comprising a reporter gene encoding a reporter, wherein expression of the reporter is regulated by LAG-3-mediated inhibition of TCR signaling within the effector T cells, and wherein expression of the reporter is altered (i.e. increased or decreased) in each effector T cell in response to activation of the effector T cell via the TCR; and

a T-cell activator capable of antigen-independent, MHC class II-independent, TCR-mediated activation of the effector T cells, wherein the T-cell activator comprises an anti-CD3 antibody, or a fragment or derivative thereof that retains antigen-independent, MHC class II-independent, TCR-mediated effector T-cell activation ability;

wherein the kit does not include a natural ligand for LAG-3, wherein the natural ligand is MHC class II.

Antigen-independent, MHC class II-independent, T cell activators are distinguished from superantigens, such as Staphylococcal enterotoxins (SEs). SEs have binding regions for MHC Class II and the TCR. They bind first to MHC Class II, then to the variable alpha or beta chain of the TCR. Interaction with the T cell receptor allows the SE to act as a wedge between the TCR and the MHC. This displaces any antigenic peptide away from the TCR and circumvents the normal mechanism for T-cell activation. SEs are, therefore MHC class II-dependent T cell activators.

Optionally the effector T cells are contacted with a concentration of the T-cell activator at which maximal inhibition of expression of the reporter occurs in the presence of an excess of an agonist of LAG-3. Use of such a concentration of the T-cell activator optimises the accuracy of the assay.

The applicant has found that the concentration of the T-cell activator at which maximal inhibition of expression of the reporter occurs in the presence of an excess of an agonist of LAG-3 is a sub-optimal concentration of the T-cell activator (i.e. a concentration which is less than a concentration of the T-cell activator at which the greatest expression of the reporter is observed in response to activation of the effector T cells by the T-cell activator in the absence of the agonist or the candidate agonist).

Optionally the T-cell activator is contacted with the effector T cells at a concentration which is less than a concentration of the T-cell activator at which the greatest expression of the reporter is observed in response to activation of the effector T cells by the T-cell activator in the absence of the agonist or the candidate agonist.

It will be appreciated that antigen-dependent stimulation expands only antigen-specific T cells, whereas antigen-independent stimulation expands up to 100% of the effector T cells.

An example of a T-cell activator capable of antigen-independent, MHC class II-independent, TCR-mediated activation of the effector T cells is an anti-CD3 antibody. Anti-CD3 antibody binds to CD3 and activates TCR complex without antigenic peptide from an APC (i.e. antigen-independent, MHC class II-independent, TCR-mediated effector T-cell stimulation) (see Figure 2(b)). Anti-CD3 antibodies can activate up to 100% of the effector T cells.

Suitable examples of an anti-CD3 antibody include OKT3 and UCHT1. Optionally the anti-CD3 antibody is OKT3.

Optionally the anti-CD3 antibody, or fragment or derivative thereof, is contacted with the effector T-cells at a concentration of ~6-30 x $10^{-12}$ M (1-4 ng/ml for whole antibody, or molar equivalent for a fragment or derivative thereof).

Optionally a kit of the invention comprises anti-CD3 antibody, or a fragment or derivative thereof that retains antigen-independent, MHC class II-independent, TCR-mediated effector T-cell activation ability, at a concentration to allow its use in the assay at a concentration of ~6-30 x $10^{-12}$ M (1-4 ng/ml for whole antibody). For example, the kit may comprise one or more aliquots of anti-CD3 antibody, or fragment or derivative thereof, at a concentration of ~6-30 x $10^{-12}$ M (1-4 ng/ml for whole antibody).

Optionally the effector T cells are contacted with several different concentrations of the agonist or the candidate agonist. For example, a plurality of different assays may be carried out in parallel, wherein the effector T cells for each different assay are contacted with a different concentration of the agonist or the candidate agonist.

Optionally an $IC_{50}$ value of the candidate agonist or agonist for inhibition of expression of the reporter is determined. This can be done, for example, from a dose-response curve generated from the results obtained by contacting the effector T cells with several different concentrations of the candidate agonist or agonist.

Optionally an assay of the invention further comprises carrying out a negative control assay (for example, in parallel with the assay). For example, the effector T cells may be contacted with the T-cell activator under conditions for antigen-independent activation of the effector T cells by the T-cell activator in the absence of the agonist or candidate agonist, but in the presence of a molecule of the same type as the agonist or candidate agonist, but which is known to lack agonist activity for LAG-3 (i.e. as a negative control).

For example, if the agonist or candidate agonist is an antibody, optionally the molecule for use as a negative control is also an antibody. Preferably the negative control antibody is an antibody of the same isotype as the agonist or candidate agonist antibody.

Optionally the T cell activator is a cell-free T-cell activator.

Optionally a kit of the invention does not comprise a cell expressing MHC class II molecules.

Optionally a kit of the invention does not comprise Raji cells.

Optionally a kit of the invention does not comprise APCs, aAPCs, or any other MHC class I-, or MHC class II-expressing cells, such as Raji cells.

Optionally the only cells in a kit of the invention are the effector T cells.

Optionally the effector T cells of a kit of the invention are frozen, for example "Thaw-and-Use" cells.

There is also provided according to the invention a kit of the invention for carrying out an assay of the invention.

Optionally the reporter gene is a heterologous reporter gene.

Genetic reporters are used widely as indicators to study gene expression and cellular events coupled to gene expression for pharmaceutical and biomedical research. Typically, a reporter gene encoding the reporter is cloned into an expression vector that is then transferred into cells. Following transfer, the cells are assayed for the presence of the reporter by directly measuring the reporter protein itself or the enzymatic activity of the reporter protein. Preferred reporters are those that can be identified easily and measured quantitatively when expressed in the effector T cells. Many suitable examples are known to those skilled in the art, including fluorescent and luminescent reporters. Optionally the reporter is a bioluminescent reporter, such as a luciferase.

Bioluminescence is a special form of chemiluminescence found in living organisms. This class of chemiluminescence is an enzyme-catalyzed process, where the high efficiency of photon emission is derived through natural evolution. The enzymes are called luciferases, and the photon-emitting substrates are luciferins. Bioluminescent chemistries have evolved from multiple independent origins and comprise many distinct molecular structures. Of the many natural forms, two have been used widely for genetic reporter assays: firefly luciferase and Renilla luciferase (Fan, and Wood (2007) Bioluminescent assays for high-throughput screening. Assay Drug Dev. Technol. 5, 127-36).

Bioluminescent reporter gene assays have a distinct advantage over fluorescent assays, such as green fluorescent protein (GFP), in that they can deliver 10- to 1,000-fold higher assay sensitivity. Both fluorescence and luminescence yield photons as a consequence of energy transitions from excited-state molecular orbitals to lower-energy orbitals. However, they differ in how the excited-state orbitals are created. In luminescence, the excited states are the product of exothermic chemical reactions, whereas in fluorescence the excited states are created by absorption of light. In reporter assays, bioluminescence is advantageous because photons are not required to create the excited states.

Therefore, they do not constitute an inherent background when measuring photon efflux from a sample. The resulting low background permits precise measurement of small changes in light across four to eight orders of magnitude in the linear range of the assay.

Luciferase reporter technology is based on the interaction of the enzyme luciferase with a luminescent substrate luciferin, which releases light by the process of bioluminescence. Bioluminescence is found in a number of different organisms; however, it is firefly (*Photinus pyralis*) luciferase that is by far the most commonly used bioluminescent reporter due to both the sensitivity and convenience of the enzyme assay and to the tight coupling of protein synthesis with enzyme activity. This monomeric enzyme of 61 kDa catalyzes a two-step oxidation reaction to yield light, usually in the green to yellow region, typically 550-570 nm. The gene encoding firefly luciferase (luc) is a cDNA and does not require any post-translational modifications. This means it is available as a mature enzyme directly upon translation from its mRNA.

Suitable reporter genes encoding luciferase are available commercially from several companies, including Promega.

By coupling operative regulatory elements to expression of a luciferase gene, typically by placing the regulatory element just upstream of a gene encoding luciferase, activation of the effector T cells can be detected readily by a luminescent signal. Typically, reporter genes are downstream of cloned response elements.

Optionally the reporter gene is under control of a promoter or response element. A suitable response element comprises a NFAT (nuclear factor of activated T cells) response element (NFAT-RE).

As explained above, TCR stimulation induces intracellular calcium release and activation of calcineurin, which dephosphorylates cytoplasmic nuclear factor of activated T cells (NFAT). Dephosphorylated NFAT translocates to the nucleus and binds to NFAT-RE, inducing transcription of the reporter gene.

Optionally the effector T cells comprise heterologous nucleic acid comprising the reporter gene.

Optionally the effector T cells comprise heterologous nucleic acid encoding LAG-3.

The term "heterologous" reporter gene or nucleic acid is used herein to include reference to a reporter gene or nucleic acid which is not naturally present in the effector T cells, but which has been introduced into the effector T cells or into effector T cells from which the effector T cells are derived, for example by cloning, recombinant technology, or transfection (techniques which are well-known to the skilled person).

Optionally the effector T cells are double-transfected with heterologous nucleic acid comprising the reporter gene (optionally wherein the reporter gene is operably linked to a promoter or response element for directing expression of the reporter gene in the effector T cell), and heterologous nucleic acid encoding LAG-3.

Optionally a kit of the invention further comprises a molecule of the same type as the agonist or candidate agonist, but which is known to lack agonist activity for LAG-3, for use as a negative control.

For example, if the agonist or candidate agonist is an antibody, optionally the molecule for use as a negative control is also an antibody. Preferably the negative control antibody is an antibody of the same isotype as the agonist or candidate agonist antibody.

Optionally a kit of the invention further comprises a known agonist of LAG-3 for use as a positive control. For example, the known agonist may be an agonist antibody, or fragment or derivative thereof that retains agonist activity.

It will be appreciated that an assay of the invention is carried out in the absence of a natural ligand for LAG-3 because the natural ligand would otherwise interfere with the results of the assay.

A kit of the invention does not include a natural ligand for LAG-3, wherein the natural ligand is MHC class II.

Effector T cells include several T cell types that actively respond to a stimulus, such as co-stimulation. They include $CD4^+$, $CD8^+$, and regulatory T cells. A suitable example is a Jurkat cell. Jurkat cells are immortalized T lymphocytes first derived from the peripheral blood of a child with T cell leukemia (Schneider et al., 1977, Int J Cancer 19 (5):621-6).

Optionally the agonist is an agonist anti-LAG-3 antibody, or a fragment or derivative thereof that retains anti-LAG-3 agonist activity.

Optionally the effector T cells comprise a T cell line that has been double-transfected with nucleic acid encoding LAG-3, and a reporter gene under the control of a promoter or response element, for example a Jurkat cell line double transfected with nucleic acid encoding LAG-3, and a NFAT/luciferase reporter gene (Jurkat LAG-$3^+$/NFAT-luc cells). Jurkat $LAG3_+$/NFAT-luc2 cells are available from Promega (ref: CS194801). Jurkat $LAG3_+$/NFAT-luc cells are available from BPS Bioscience (Catalog #: 71278).

Optionally the agonist is an agonist anti-LAG-3 antibody, or a fragment or derivative thereof that retains anti-LAG-3 agonist activity, and the effector T cells comprise Jurkat LAG-$3^+$/NFAT-luc2 cells.

Optionally the agonist anti-LAG-3 antibody is an agonist anti-LAG-3 antibody described in WO 2017/037203. Agonist anti-LAG-3 antibodies decribed in WO 2017/037203 include mouse monoclonal antibody 13E2, and humanized 13E2-human IgG4 Fc antibody (referred to as IMP761). Antibodies 13E2 and IMP761 comprise VH CDR1-3 and VL CDR1-3 sequences as set out in Table 1 below:

Table 1. Anti-LAG-3 Agonist Antibody VH and VL CDR Sequences

| **Antibody : 13E2 ; IMP761** | **CDR-1** | **CDR-2** | **CDR-3** |
|---|---|---|---|
| **VH** (IMGT numbering) | GFSLSTSGMG (SEQ ID NO:1) | IWWDDIK (SEQ ID NO:2) | ARIVEGSYSSSYFDV (SEQ ID NO:3) |
| **VL** (IMGT numbering) | QDVIFD (SEQ ID NO:4) | SAS (SEQ ID NO:5) | QQHYSTPYT (SEQ ID NO:6) |
| **VH** (Kabat numbering) | TSGMGLG (SEQ ID NO:7) | HIWWDDIKRYNPDLRS (SEQ ID NO:8) | IVEGSYSSSYFDV (SEQ ID NO:9) |
| **VL** (Kabat numbering) | KASQDVIFDVA (SEQ ID NO:10) | SASSRVS (SEQ ID NO:11) | QQHYSTPYT (SEQ ID NO:12) |

Optionally the agonist anti-LAG-3 antibody, or fragment or derivative thereof, comprises VH CDR1-3 and VL CDR1-3 sequences of SEQ ID NOs:1-6, respectively, or VH CDR1-3 and VL CDR1-3 sequences of SEQ ID Nos:7-12, respectively:

Optionally the agonist anti-LAG-3 antibody is IMP761.

Embodiments of the invention are described below, by way of example only, with reference to the accompanying drawings in which:

Figure 1 shows a schematic representation of TCR signaling (from Belikov, Aleksey. (2016). The role of reactive oxygen species and mitochondria in T-cell activation. 10.13140/RG.2.1.2916.0568);

Figure 2 shows the mode of action of an IMP761 potency assay according to an embodiment of the invention. In the presence of anti-CD3 antibody, the anti-CD3 antibody binds to CD3 on the surface of the Reporter Jurkat Cell, leading to increased expression of luciferase from the NFAT/Luc reporter gene through TCR-mediated signal transduction (upper part). Binding of IMP761 to LAG-3 on the surface of the Reporter Jurkat Cell inhibits TCR-mediated signal transduction, leading to downregulation of expression of luciferase from the NFAT/Luc reporter gene (lower part);

Figure 3 shows the effect of stimulation of Jurkat LAG-$3_+$/NFAT-luc2 cells by different concentrations of OKT3 and UCHT1 anti-CD3 antibody in the presence of 300ng/ml IMP761, or human IgG4 negative control antibody; and

Figure 4 shows an example of results of a potency assay for IMP761 versus an IgG4 negative control, according to an embodiment of the invention; and

Figure 5 shows an example of results of a potency assay with a reference preparation of IMP761 (4°C), compared to a preparation of IMP761 after denaturating temperature stress (10 or 20 minutes at 70°C), according to an embodiment of the invention.

## Example 1

### Optimization of IMP761 Potency Assay Protocol

Jurkat Lag-$3_+$/NFAT-luc2 effector cells were initially developed by Promega to determine antagonist anti-LAG-3 antibody activity after TCR activation through superantigen stimulation presented by MHC II molecules. Antagonist anti-LAG-3 antibody blockage of the LAG-3/MHC II interaction results in enhanced TCR activation and luciferase activity. The use of Jurkat Lag-$3_+$/NFAT-luc2 effector cells to determine agonist anti-LAG-3 antibody activity requires a very different experimental set-up, as explained below.

Anti-CD3 antibody as a stimulator of Jurkat cells:

In the Promega bioassay, Jurkat Lag-$3_+$/NFAT-luc2 effector cells are activated using Raji cells in the presence of Staphylococcal Entereotoxin E or D (SEE or SED). Raji cells express endogenous MHC class II, a LAG-3 ligand. This is important to test the blocking activity of antagonist anti-LAG-3 antibody on the LAG-3/MHC II interaction. However, as no LAG-3/MHC II interaction is required for testing the potency of an agonist anti-LAG-3 antibody, there is no requirement for Raji cells nor the Staphylococcal Enterotoxin. A single cell-type assay was used with anti-CD3 antibodies to activate Jurkat Lag-$3_+$/NFAT-luc2 effector cells through TCR signaling.

Anti-CD3 concentration and LAG-3-related inhibition:

The effect on the cell potency assay of two different anti-CD3 antibodies (OKT3 and UCHT1) was tested at different antibody concentrations, ranging from 1 to 500 ng/ml.

Jurkat Lag-$3_+$/NFAT-luc2 cells were incubated with 300 ng/ml of IMP761, or human-IgG4 (as a negative control), in the presence of different concentrations of OKT3 or UCHT1 for 24 hours. The average RLU values obtained for the different concentrations of anti-CD3 antibody are shown in Figure 3, and in Table 2 below:

Table 2

| | | [anti CD3] ng/ml | | | | | | | | | |
|---|---|---|---|---|---|---|---|---|---|---|---|
| | | **0** | **1.95** | **3.91** | **7.81** | **15.63** | **31.25** | **62.50** | **125.00** | **250.00** | **500** |
| OKT3 | hIgG4 | 209832 | 469467 | 889256 | 1862472 | 3067845 | 4011104 | 4083312 | 2786384 | 2087877 | 1652699 |
| | IMP761 | 51635 | 89112 | 190253 | 597080 | 1399133 | 2178685 | 2124312 | 1405789 | 1086563 | 1073739 |
| UCHT1 | hIgG4 | 131512 | 138995 | 145635 | 178997 | 256723 | 497907 | 730163 | 814064 | 806883 | 827064 |
| | IMP761 | 56024 | 49957 | 47856 | 58803 | 79861 | 126859 | 228299 | 319683 | 453048 | 540205 |

Luciferase activty is inhibited by IMP761 across the range of different concentrations of each anti-CD3 antibody.

There is a basal level of reporter expression in the Jurkat cell line without stimulation, so luciferase activity and inhibition of luciferase activity by IMP761 was also tested in the absence of anti-CD3 antibody. However, stimulation of the Jurkat cells with anti-CD3 antibody gives higher RLU values, so the inhibitory effect of IMP761 is more apparent in the presence of anti-CD3 antibody (in particular, a low concentration of anti-CD3 antibody). The percentage inhibition of luciferase activity for OKT3 antibody is shown in Table 3 below:

Table 3

| anti-CD3 concentration (ng/ml) | % Inhibition |
|---|---|
| 0 | 75.4 |
| 1.95 | 81.0 |
| 3.91 | 78.6 |
| 7.81 | 67.9 |
| 15.63 | 54.4 |
| 31.25 | 45.7 |
| 62.5 | 48.0 |
| 125 | 49.5 |
| 250 | 48.0 |
| 500 | 35.0 |

It was concluded that the maximum effect of IMP761 (inhibition of approximately 80%) was observed when a low concentration of OKT3 antibody (between 1 and 4 ng/ml) was used. Thus, an optimum potency assay includes stimulation of Jurkat cells with low concentrations of anti-CD3 antibody (for example, OKT3 antibody).

## Example 2

### IMP761 Potency Assay

This example describes a potency assay, according to an embodiment of the invention, to measure the activity of the IMP761 monoclonal antibody *in vitro.* The method is based on the capacity of IMP761 to decrease the activation of a LAG-3 effector cell line induced by a low dose of an anti-CD3 antibody (OKT3 clone, 3 ng/ml), mimicking antigen-stimulation of T cells. The LAG-3 effector cell line is a Jurkat T cell line expressing LAG-3 at the surface, and containing the luciferase gene under the control of an NFAT (nuclear factor of activated T cells) response element *(Jurkat Lag-3$^+$/NFAT-luc2 effector cells,* from Promega). After binding to its target, IMP761 triggers a downregulation of TCR-induced NFAT-regulated expression (illustrated schematically in Figure 2, lower part). Luciferase activity of the cell line is used to measure TCR-driven cell activation which is dampened in the presence of IMP761 activity. Thus, the assay measures IMP761 potency in terms of its ability to inhibit TCR signaling.

Reagents

Jurkat LAG-$3_+$/NFAT-luc2 effector cells (Promega, ref: CS194801)
RPMI 1640 (GIBCO, ref: 31870-025)
L-Glutamine (200 mM) (GIBCO, ref: 25030-024)
HEPES (1 M) (GIBCO, ref: 15630-080)
FCS (GIBCO, ref: 10270106)
IMP761 (2.06 mg/ml) (IMMUTEP, lot: 270416)
human IgG4, control (Biolegend, ref: 403402)
Anti-CD3 (OKT3) (eBioscience, ref: 16-0037-85)
Bio-Glo reagent (PROMEGA, ref: G7941)
White, 96-well solid flat-bottom microplates (COSTAR, ref: 3917)
Assay medium: RPMI 1640, L-Glutamine (2 mM), Hepes (10 mM), FCS 1%

Cell concentration: 1.33 x $10^6$ cells/ml

Protocol

1. Pass Jurkat LAG-$3_+$/NFAT-luc2 effector cells at day -1 before assay in order to have a cell density around 1 Million/ml (between 0.8 and 1.2 Million/ml) at day 0.
2. Prepare assay medium if needed and pre-warm medium for 30 minutes at 37°C:

| | |
|---|---|
| FCS | 0.5 ml |
| L-Glutamine (200mM) | 0.5 ml |
| HEPES (1M) | 0.5 ml |
| RPMI 1640 | 48.5 ml |

3. Prepare IMP761 quality control (QC) stock solution if needed:

Make 24,000 ng/ml stock solution; for example:

10µl of stock IMP761 (2.06 mg/ml) + 848.3µl Assay medium.

Store 25 µl aliquots in -80°C freezer.

4. Prepare 3X solutions of IMP761 (batch 270416 at 2.06 mg/ml), human IgG4 negative control (or any other antibody). Adapt the predilution steps according to the initial concentration of the antibody: the volume of assay medium (in µl) to be added to dilute 2 µl of a stock solution in order to prepare a 100 µg/ml predilution is:

$$\left(2\ x\left(\frac{Initial\ concentration\ (in\ mg/ml)}{0.1}\right)-2\right)\ \mu l\ of\ assay\ medium$$

| | IMP761 Dilution to prepare 3X working solutions | | |
|---|---|---|---|
| | Predilution (100 µg/ml) | 2 µl of stock (2.06 mg/ml) + 39.2 µl of assay medium | Final concentration (ng/ml) |
| Serial Dilutions | 2000 ng/ml | 10 µl of 100 µg/ml predilution + 490 µl of assay medium | 666.7 |
| | 1000ng/ml | 200 µl of 2000 ng/ml dilution + 200 µl of assay medium | 333.3 |
| | 500 ng/ml | 200 µl of 1000 ng/ml dilution + 200 µl of assay medium | 166.7 |
| | 250 ng/ml | 200 µl of 500 ng/ml dilution + 200 µl of assay medium | 83.3 |
| | 125 ng/ml | 200 µl of 250 ng/ml dilution + 200 µl of assay medium | 41.7 |
| | 62.5 ng/ml | 200 µl of 125 ng/ml dilution + 200 µl of assay medium | 20.8 |
| | 31.2ng/ml | 200 µl of 62.5 ng/ml dilution + 200 µl of assay medium | 10.4 |
| | 15.6ng/ml | 200 µl of 31.2 ng/ml dilution + 200 µl of assay medium | 5.2 |
| | 7.8 ng/ml | 200 µl of 15.6 ng/ml dilution + 200 µl of assay medium | 2.6 |

5. Prepare 3X IMP761 quality control (QC) solutions
Thaw stock IMP761 QC [24,000 ng/ml] aliquot and make serial dilutions

| | | |
|---|---|---|
| Very High (VH) QC: | 20 µl | [24,000 ng/ml] + 380 µl = 1,200 ng/ml (final concentration: 400 ng/ml) |
| High (H) QC: | 150 µl | [1,200 ng/ml] + 225 µl = 480 ng/ml (final concentration: 160 ng/ml) |
| Medium (M) QC: | 150 µl | [480 ng/ml] + 225 µl = 192 ng/ml (final concentration: 64 ng/ml) |

(continued)

| | | |
|---|---|---|
| Low (L) QC: | 150 µl | [192 ng/ml] + 225 µl = 76.8 ng/ml (final concentration: 25.6 ng/ml) |
| Very Low (VL) QC: | 150 µl | [25.6 ng/ml] + 225 µl = 10.24 ng/ml (final concentration: 3.4 ng/ml) |

6. Prepare OKT3 reagent

$$3.6\ \mu l \text{ stock} + 4\ ml \text{ assay medium} = 0.9\ \mu g/ml$$

$$150\ \mu l \text{ of } [0.9\ \mu g/ml] + 14{,}850\ \mu l \text{ of assay medium} = 9\ ng/ml\ (3X)$$

7. Prepare Jurkat LAG-3/NFAT-luc2 effector cells

At day 0, count cells using Trypan Blue staining
Centrifuge cells at 1200 rpm for 5 minutes
Aspirate media and resuspend cells at 3.75 x $10^6$/ml in assay medium.

8. Distribute 3X solutions in 96 well plate
Do not use outer wells because of edge effect
Distribute 40 µl of 3X solutions of IMP761 / QC / assay medium (0) into the corresponding wells of the assay plate in duplicate
Distribute 40 µl/well of 3X solution of anti-CD3 / assay medium (Unstimulated control: unstim) into each well of the assay plate in duplicate. Final concentration: 3 ng/ml
Distribute 40 µl per well (0.15 x $10^6$/well)
Example of plate template (Reference batch/unknown)

| | | | | | | | | | | | |
|---|---|---|---|---|---|---|---|---|---|---|---|
| | | | | | | | | | | | |
| | 666.7 | 333.3 | 166.7 | 83.3 | 41.7 | 20.8 | 10.4 | 5.2 | 2.6 | 0 | |
| | 666.7 | 333.3 | 166.7 | 83.3 | 41.7 | 20.8 | 10.4 | 5.2 | 2.6 | 0 | |
| | 666.7 | 333.3 | 166.7 | 83.3 | 41.7 | 20.8 | 10.4 | 5.2 | 2.6 | 0 | |
| | 666.7 | 333.3 | 166.7 | 83.3 | 41.7 | 20.8 | 10.4 | 5.2 | 2.6 | 0 | |
| | QC VHIGH | QC HIGH | QC MID | QC LOW | QC VLOW | UNSTIM | | | | | |
| | QC VHIGH | QC HIGH | QC MID | QC LOW | QC VLOW | UNSTIM | | | | | |
| | | | | | | | | | | | |

9. Incubate plate(s) at 37°C in a humidified incubator, with 5% $CO_2$, for 24 hours
10. Prepare BioGlo reagent

a) Place frozen BioGlo reagent at room temperature (RT) 3-6 hours before use to allow thawing

b) Transfer the buffer (10ml) to substrate bottle, mix and keep at RT in the dark until use

11. Equilibrate plate(s) at RT for 15 minutes
12. Add 120µl / well of BioGlo, avoid / remove bubbles
13. Incubate at RT for 5 to 15 minutes
14. Measure luminescence (RLU), integration time = 0.5 sec/well using PerkinElmer 2103 Multilabel reader Envision. 3 measures for each well are collected 45 seconds apart, calculate the average of 3 measures to obtain "RLU Average".

Results

Figure 4 shows an example of results obtained from the potency assay, using IgG4 antibody as a negative control. Maximum activation was recorded as the activation observed when no IMP761 was present. Maximum inhibition was recorded as the activation observed when IMP761 concentration was 1000 ng/ml. A five-parameter non-linear regression model determined that the $IC_{50}$ of the IMP761 was 37 ng/ml.

**Example 3**

**Ability of IMP761 potency assay to evaluate denatured IMP761 antibody**

The capacity of the potency assay as described in Example 2 to evaluate the decreased efficacy of a denatured IMP761 antibody was tested by comparing the $IC_{50}$ of a reference IMP761 stored at 4°C with the $IC_{50}$ of the same batch of IMP761 after temperature stress (10 minutes at 70°C and 20 minutes at 70°C). The results are shown in Figure 5, and in Table 4 below:

Table 4: dataset (mean of duplicates) for a potency assay with a reference IMP761 (4°C) and IMP761 after denaturing temperature stress (10 and 20 minutes at 70°C), expressed as relative light units (RLU)

| | | 70°C | |
|---|---|---|---|
| **[IMP761] ng/ml** | **4°C** | **10 minutes** | **20 minutes** |
| **667** | 141272 | 139814 | 155807 |
| **333** | 139620 | 148490 | 163200 |
| **167** | 152260 | 178717 | 188419 |
| **83** | 196259 | 271611 | 292410 |
| **56** | 274748 | 365493 | 368244 |
| **37** | 316679 | 381826 | 425702 |
| **25** | 338896 | 420090 | 429251 |
| **12** | 409866 | 441327 | 430144 |
| **6.2** | *328883** | 426592 | 450974 |
| **0** | 457495 | 468616 | 420222 |

** Outliers, removed from dataset*

A five-parameter non-linear regression model determined that the $IC_{50}$ of the denatured IMP761 antibodies (74 ng/ml and 81.5 ng/ml after 10 minutes and 20 minutes at 70°C, respectively) was higher than the $IC_{50}$ (41 ng/ml) of the reference IMP761 antibody stored at 4°C.

## Claims

1. An *in vitro* assay for determining activity of an agonist of lymphocyte-activation gene 3 (LAG-3), which comprises:

   providing a plurality of effector T cells, each effector T cell expressing LAG-3 and a T-cell receptor (TCR) on its surface, and comprising a reporter gene encoding a reporter, wherein expression of the reporter is regulated by LAG-3-mediated inhibition of TCR signaling within the effector T cells; and
   determining the activity of the agonist from the extent to which expression of the reporter is altered in the presence of the agonist compared with expression of the reporter in the absence of the agonist;
   wherein the assay is carried out in the absence of a natural ligand for LAG-3, wherein the natural ligand is MHC class II.

2. An assay according to claim 1, for determining the potency of a preparation of an agonist of LAG-3.

3. An *in vitro* assay for screening for an agonist of LAG-3, which comprises:

   providing a plurality of effector T cells, each effector T cell expressing LAG-3 and a T-cell receptor (TCR) on its surface, and comprising a reporter gene encoding a reporter, wherein expression of the reporter is regulated by LAG-3-mediated inhibition of TCR signaling within the effector T cells; and
   determining whether a candidate agonist is an agonist of LAG-3 by determining the extent to which expression of the reporter is altered in the presence of the candidate agonist compared with expression of the reporter in the absence of the candidate agonist;
wherein the assay is carried out in the absence of a natural ligand for LAG-3, wherein the natural ligand is MHC class II.

**4.** An assay according to claim 1 or 2, wherein the reporter is expressed at a basal level in the effector T cells in the absence of the agonist, or an assay according to claim 3, wherein the reporter is expressed at a basal level in the effector T cells in the absence of the candidate agonist.

**5.** An assay according to any preceding claim, wherein expression of the reporter is reduced in the presence of the agonist, or candidate agonist, compared with expression of the reporter in the absence of the agonist, or candidate agonist.

**6.** An assay according to any preceding claim, wherein expression of the reporter is altered in each effector T cell in response to activation of the effector T cell via the TCR, and which further comprises:

activating the effector T cells by antigen-independent, MHC class II-independent, TCR-mediated T-cell activation in the presence and absence of the agonist, or candidate agonist, wherein the effector T cells are activated by contacting the effector T cells with an antigen-independent, MHC class II-independent, T-cell activator under conditions for antigen-independent, MHC class II-independent, TCR-mediated activation of the effector T cells by the T-cell activator, and wherein the T-cell activator comprises or consists of an anti-CD3 antibody, or a fragment or derivative thereof that retains antigen-independent, MHC class II-independent, TCR-mediated effector T-cell activation ability; and
determining the activity of the agonist, or the candidate agonist, from the extent to which expression of the reporter, in response to activation of the effector T cells, is altered in the presence of the agonist, or candidate agonist, compared with expression of the reporter, in response to activation of the effector T cells, in the absence of the agonist, or candidate agonist; optionally
wherein expression of the reporter is increased in each effector T cell in response to activation of the effector T cell via the TCR, and is reduced in the presence of the agonist, or candidate agonist, as a result of LAG-3-mediated inhibition of TCR signaling within the effector T cell, and wherein the activity of the agonist, or candidate agonist, is determined from the extent to which expression of the reporter, in response to activation of the effector T cells, is reduced in the presence of the agonist, or candidate agonist, compared with expression of the reporter, in response to activation of the effector T cells, in the absence of the agonist, or candidate agonist.

**7.** An assay according to claim 6, wherein the effector T cells are contacted with the T-cell activator at a concentration of the T-cell activator at which maximal inhibition of expression of the reporter occurs in the presence of an excess of an agonist of LAG-3.

**8.** An assay according to claim 7, wherein the effector T cells are contacted with the T-cell activator at a concentration of the T-cell activator which is less than a concentration of the T-cell activator at which the greatest expression of the reporter is observed in response to activation of the effector T cells by the T-cell activator in the absence of the agonist, or candidate agonist.

**9.** An assay according to any of claims 6 to 8, wherein the anti-CD3 antibody is OKT3, and/or wherein the anti-CD3 antibody, or fragment or derivative thereof, is contacted with the effector T-cells at a concentration of ~6-30 x $10^{-12}$M (1-4 ng/ml for whole antibody, or molar equivalent for fragment or derivative thereof).

**10.** An assay according to any preceding claim, wherein the effector T-cells are activated by cell-free, antigen-independent, MHC class II-independent, TCR-mediated T-cell activation, wherein the effector T cells are activated by contacting the effector T cells with an antigen-independent, MHC class II-independent, T-cell activator under conditions for antigen-independent, MHC class II-independent, TCR-mediated activation of the effector T cells by the T-cell activator, and wherein the T-cell activator comprises or consists of an anti-CD3 antibody, or a fragment or derivative thereof that retains antigen-independent, MHC class II-independent, TCR-mediated effector T-cell activation ability, and/or wherein the effector T cells are contacted with several different concentrations of the agonist or candidate agonist, optionally wherein the assay further comprises determining an $IC_{50}$ value of the agonist, or candidate agonist, for inhibition of expression of the reporter.

**11.** An assay according to any preceding claim, wherein:

the effector T cells comprise heterologous nucleic acid comprising the reporter gene; and/or
the reporter gene is under control of a promoter or response element, preferably wherein the response element comprises a NFAT (nuclear factor of activated T cells) response element (NFAT-RE); and/or
the reporter comprises a bioluminescent reporter, such as a luciferase; and/or the effector T cells comprise heterologous nucleic acid encoding LAG-3; and/or
the assay further comprises carrying out a negative control assay, wherein the effector T cells are activated in the absence of the agonist or candidate agonist, but in the presence of a molecule of the same type as the agonist or candidate agonist, but which is known to lack agonist activity for LAG-3; and/or the agonist or candidate agonist is an anti-LAG-3 antibody, or a fragment or derivative thereof that retains anti-LAG-3 agonist activity; and/or
the effector T cells comprise Jurkat-derived cells; and/or
the agonist is an agonist anti-LAG-3 antibody, or a fragment or derivative thereof that retains anti-LAG-3 agonist activity, and the effector T cells comprise Jurkat LAG-3$^+$/NFAT-luc2 cells; and/or
the agonist is an anti-LAG-3 antibody, or a fragment or derivative thereof, which comprises VH CDR1-3 sequences and VL CDR1-3 sequences of SEQ ID NOs:1-6, respectively, or SEQ ID NOs:7-12, respectively; and/or
the agonist is anti-LAG-3 antibody IMP761.

**12.** A kit for carrying out an *in vitro* assay for determining activity of, or screening for, an agonist of LAG-3, which comprises:

a plurality of effector T cells, each effector T cell expressing LAG-3 and a T-cell receptor (TCR) on its surface, and comprising a reporter gene encoding a reporter, wherein expression of the reporter is regulated by LAG-3-mediated inhibition of TCR signaling within the effector T cells, and wherein expression of the reporter is altered in each effector T cell in response to activation of the effector T cell via the TCR; and
a T-cell activator capable of antigen-independent, MHC class II-independent, TCR-mediated activation of the effector T cells, wherein the T-cell activator comprises an anti-CD3 antibody, or a fragment or derivative thereof that retains antigen-independent, MHC class II-independent, TCR-mediated effector T-cell activation ability;
wherein the kit does not include a natural ligand for LAG-3, wherein the natural ligand is MHC class II.

**13.** A kit according to claim 12, wherein:

the kit does not comprise a cell expressing MHC class II molecules; and/or
expression of the reporter is increased in response to activation of the effector T cells via the TCR; and/or
the effector T cells comprise heterologous nucleic acid comprising the reporter gene; and/or
the reporter gene is under control of a promoter or response element, preferably wherein the response element comprises a NFAT (nuclear factor of activated T cells) response element (NFAT-RE); and/or
the reporter comprises a bioluminescent reporter, such as a luciferase; and/or
the effector T cells comprise heterologous nucleic acid encoding LAG-3; and/or
the T-cell activator is a cell-free T-cell activator; and/or
the anti-CD3 antibody is OKT3; and/or
the anti-CD3 antibody, or fragment or derivative thereof, is present at a concentration to allow its use in the assay at a concentration of ~6-30 x $10^{-12}$ M (1-4 ng/ml for whole antibody, or molar equivalent for fragment or derivative thereof); and/or
the kit further comprises a molecule of the same type as the agonist, but which is known to lack agonist activity for LAG-3, for use as a negative control; and/or
the kit further comprises a known agonist of LAG-3 for use as a positive control; and/or
the effector T cells comprise Jurkat-derived cells; and/or
the effector T cells comprise Jurkat LAG-3$^+$/NFAT-luc2 cells; and/or
the kit does not comprise APCs, aAPCs, or any other MHC class I-, or MHC class II-expressing cells; and/or
the only cells in the kit are the effector T cells.

**14.** A kit according to claim 12 or 13, for determining the potency of a preparation of an agonist of LAG-3.

**15.** A kit according to claim 12 or 13 for carrying out an assay according to any of claims 1 to 11.

**16.** Use of a kit according to claim 12 or 13 for determining activity of an agonist of LAG-3, determining the potency of a preparation of an agonist of LAG-3, or screening for an agonist of LAG-3.

**Patentansprüche**

1. *In-vitro-Assay* zur Bestimmung der Aktivität eines Agonisten des Lymphozyten-Aktivierungsgens 3 (LAG-3), das Folgendes umfasst:

   Bereitstellen einer Mehrzahl von Effektor-T-Zellen, wobei jede Effektor-T-Zelle LAG-3 und einen T-Zell-Rezeptor (TCR) auf ihrer Oberfläche exprimiert und ein Reportergen umfasst, das einen Reporter kodiert, wobei die Expression des Reporters durch LAG-3-vermittelte Inhibition der TCR-Signalisierung in den Effektor-T-Zellen reguliert wird; und
   Bestimmen der Aktivität des Agonisten aus dem Ausmaß, in dem die Expression des Reporters in Anwesenheit des Agonisten im Vergleich zur Expression des Reporters in Abwesenheit des Agonisten verändert ist;
   wobei der Assay in Abwesenheit eines natürlichen Liganden für LAG-3 durchgeführt wird, wobei der natürliche Ligand MHC-Klasse II ist.

2. Assay nach Anspruch 1 zur Bestimmung der Wirkstärke eines Präparats eines Agonisten von LAG-3.

3. *In-vitro-Assay* zum Screening auf einen LAG-3-Agonisten, das Folgendes beinhaltet:

   Bereitstellen einer Mehrzahl von Effektor-T-Zellen, wobei jede Effektor-T-Zelle LAG-3 und einen T-Zell-Rezeptor (TCR) auf ihrer Oberfläche exprimiert und ein Reportergen umfasst, das einen Reporter kodiert, wobei die Expression des Reporters durch LAG-3-vermittelte Inhibition der TCR-Signalisierung in den Effektor-T-Zellen reguliert wird; und
   Feststellen, ob ein Agonistenkandidat ein LAG-3-Agonist ist, durch Bestimmen des Ausmaßes, in dem die Expression des Reporters in Anwesenheit des Agonistenkandidaten im Vergleich zur Expression des Reporters in Abwesenheit des Agonistenkandidaten verändert ist;
   wobei das Assay in Abwesenheit eines natürlichen Liganden für LAG-3 durchgeführt wird, wobei der natürliche Ligand MHC-Klasse II ist.

4. Assay nach Anspruch 1 oder 2, wobei der Reporter in den Effektor-T-Zellen in Abwesenheit des Agonisten auf einem basalen Niveau exprimiert wird, oder Assay nach Anspruch 3, wobei der Reporter in den Effektor-T-Zellen in Abwesenheit des Agonistenkandidaten auf einem basalen Niveau exprimiert wird.

5. Assay nach einem vorherigen Anspruch, wobei die Expression des Reporters in Anwesenheit des Agonisten oder Agonistenkandidaten im Vergleich zur Expression des Reporters in Abwesenheit des Agonisten oder Agonistenkandidaten reduziert ist.

6. Assay nach einem vorherigen Anspruch, wobei die Expression des Reporters in jeder Effektor-T-Zelle als Reaktion auf die Aktivierung der Effektor-T-Zelle über den TCR verändert wird, und der ferner Folgendes umfasst:

   Aktivieren der Effektor-T-Zellen durch antigenunabhängige, MHC-Klasse-II-unabhängige, TCR-vermittelte T-Zell-Aktivierung in An- und Abwesenheit des Agonisten oder Agonistenkandidaten, wobei die Effektor-T-Zellen durch Inkontaktbringen der Effektor-T-Zellen mit einem antigenunabhängigen, MHC-Klasse-II-unabhängigen T-Zell-Aktivator unter Bedingungen für eine antigenunabhängige, MHC-Klasse-II-unabhängige, TCR-vermittelte Aktivierung der Effektor-T-Zellen durch den T-Zellaktivator aktiviert werden, und wobei der T-Zell-Aktivator einen Anti-CD3-Antikörper oder ein Fragment oder Derivat davon umfasst oder daraus besteht, der/das die Fähigkeit zur antigenunabhängigen, MHC-Klasse-II-unabhängigen, TCR-vermittelten Aktivierung von Effektor-T-Zellen beibehält; und
   Bestimmen der Aktivität des Agonisten oder des Agonistenkandidaten aus dem Ausmaß, in dem die Expression des Reporters als Reaktion auf die Aktivierung der Effektor-T-Zellen in Anwesenheit des Agonisten oder Agonistenkandidaten im Vergleich zur Expression des Reporters als Reaktion auf die Aktivierung der Effektor-T-Zellen in Abwesenheit des Agonisten oder Agonistenkandidaten verändert ist; wobei optional
   die Expression des Reporters in jeder Effektor-T-Zelle als Reaktion auf die Aktivierung der Effektor-T-Zelle über den TCR erhöht ist und in Anwesenheit des Agonisten oder Agonistenkandidaten als Ergebnis der LAG-3-vermittelten Inhibition der TCR-Signalisierung in der Effektor-T-Zelle verringert ist, und wobei die Aktivität des Agonisten oder Agonistenkandidaten aus dem Ausmaß bestimmt wird, in dem die Expression des Reporters als Reaktion auf die Aktivierung der Effektor-T-Zellen in Anwesenheit des Agonisten oder Agonistenkandidaten im Vergleich zur Expression des Reporters als Reaktion auf die Aktivierung der Effektor-T-Zellen in Abwesenheit des Agonisten oder Agonistenkandidaten reduziert ist.

**7.** Assay nach Anspruch 6, wobei die Effektor-T-Zellen mit dem T-Zell-Aktivator in einer Konzentration des T-Zell-Aktivators in Kontakt gebracht werden, bei der eine maximale Inhibition der Expression des Reporters in Anwesenheit eines Überschusses eines LAG-3-Agonisten auftritt.

**8.** Assay nach Anspruch 7, wobei die Effektor-T-Zellen mit dem T-Zell-Aktivator bei einer Konzentration des T-Zell-Aktivators in Kontakt gebracht werden, die geringer ist als eine Konzentration des T-Zell-Aktivators, bei der die größte Expression des Reporters als Reaktion auf die Aktivierung der Effektor-T-Zellen durch den T-Zell-Aktivator in Abwesenheit des Agonisten oder Agonistenkandidaten beobachtet wird.

**9.** Assay nach einem der Ansprüche 6 bis 8, wobei der Anti-CD3-Antikörper OKT3 ist und/oder wobei der Anti-CD3-Antikörper oder ein Fragment oder Derivat davon mit den Effektor-T-Zellen bei einer Konzentration von ~6-30 x $10^{-12}$ M (1-4 ng/ml für den gesamten Antikörper oder das molare Äquivalent für das Fragment oder Derivat davon) in Kontakt gebracht wird.

**10.** Assay nach einem vorherigen Anspruch, wobei die Effektor-T-Zellen durch zellfreie, antigenunabhängige, MHC-Klasse-II-unabhängige, TCR-vermittelte T-Zell-Aktivierung aktiviert werden, wobei die Effektor-T-Zellen durch Inkontaktbringen der Effektor-T-Zellen mit einem antigenunabhängigen, MHC-Klasse-II-unabhängigen T-Zell-Aktivator unter Bedingungen für antigenunabhängige, MHC-Klasse-II-unabhängige TCR-vermittelte Aktivierung der Effektor-T-Zellen durch den T-Zell-Aktivator aktiviert werden, und wobei der T-Zell-Aktivator einen Anti-CD3-Antikörper oder ein Fragment oder Derivat davon umfasst oder daraus besteht, der/das die Fähigkeit zur antigenunabhängigen, MHC-Klasse-II-unabhängigen, TCR-vermittelten Effektor-T-Zell-Aktivierung beibehält, und/oder wobei die Effektor-T-Zellen mit mehreren unterschiedlichen Konzentrationen des Agonisten oder Agonistenkandidaten in Kontakt gebracht werden, wobei optional das Assay ferner die Bestimmung eines $IC_{50}$-Werts des Agonisten oder Agonistenkandidaten zur Inhibition der Expression des Reporters umfasst.

**11.** Assay nach einem vorherigen Anspruch, wobei:

die Effektor-T-Zellen eine heterologe Nukleinsäure umfassen, die das Reportergen umfasst; und/oder
das Reportergen unter der Kontrolle eines Promotor- oder Antwortelements steht, wobei das Antwortelement vorzugsweise ein NFAT (nuclear factor of activated T cells)-Antwortelement (NFAT-RE) umfasst; und/oder
der Reporter einen biolumineszenten Reporter, wie eine Luciferase, umfasst; und/oder die Effektor-T-Zellen heterologe Nukleinsäure umfassen, die LAG-3 kodiert; und/oder
das Assay ferner die Durchführung eines Negativkontrollassay umfasst, wobei die Effektor-T-Zellen in Abwesenheit des Agonisten oder Agonistenkandidaten, aber in Anwesenheit eines Moleküls des gleichen Typs wie der Agonist oder Agonistenkandidat aktiviert werden, dem jedoch bekanntlich Agonistenaktivität für LAG-3 fehlt; und/oder der Agonist oder Agonistenkandidat ein Anti-LAG-3-Antikörper oder ein Fragment oder Derivat davon ist, das die Anti-LAG-3-Agonistenaktivität beibehält; und/oder
die Effektor-T-Zellen von Jurkat abgeleitete Zellen umfassen; und/oder
der Agonist ein agonistischer Anti-LAG-3-Antikörper oder ein Fragment oder Derivat davon ist, das die Anti-LAG-3-Agonistenaktivität beibehält, und die Effektor-T-Zellen Jurkat-LAG-$3^+$/NFAT-luc2-Zellen umfassen; und/oder
der agonistische Anti-LAG-3-Antikörper oder ein Fragment oder Derivat davon ist, das VH-CDR1-3-Sequenzen und VL-CDR1-3-Sequenzen von SEQ ID NOs:1-6 bzw. SEQ ID NOs:7-12 umfasst; und/oder
der agonistische Anti-LAG-3-Antikörper IMP761 ist.

**12.** Kit zur Durchführung eines *In-vitro*-Assays zur Bestimmung der Aktivität eines LAG-3-Agonisten oder zum Screening auf einen solchen, der Folgendes umfasst:

eine Mehrzahl von Effektor-T-Zellen, wobei jede Effektor-T-Zelle LAG-3 und einen T-Zell-Rezeptor (TCR) auf ihrer Oberfläche exprimiert und ein Reportergen umfasst, das einen Reporter kodiert, wobei die Expression des Reporters durch LAG-3-vermittelte Inhibition der TCR-Signalisierung in den Effektor-T-Zellen reguliert wird, und wobei die Expression des Reporters in jeder Effektor-T-Zelle als Reaktion auf die Aktivierung der Effektor-T-Zelle über den TCR verändert wird; und
einen T-Zell-Aktivator, der zu einer antigenunabhängigen, MHC-Klasse-II-unabhängigen, TCR-vermittelten Aktivierung der Effektor-T-Zellen fähig ist, wobei der T-Zell-Aktivator einen Anti-CD3-Antikörper oder ein Fragment oder Derivat davon umfasst, der/das die Fähigkeit zur antigenunabhängigen, MHC-Klasse-II-unabhängigen, TCR-vermittelten Aktivierung von Effektor-T-Zellen beibehält;
wobei der Kit keinen natürlichen Liganden für LAG-3 enthält, wobei der natürliche Ligand MHC-Klasse-II ist.

**13.** Kit nach Anspruch 12, wobei:

das Kit keine Zelle umfasst, die MHC-Klasse-II-Moleküle exprimiert; und/oder
die Expression des Reporters als Reaktion auf die Aktivierung der Effektor-T-Zellen über den TCR erhöht ist; und/oder
die Effektor-T-Zellen eine heterologe Nukleinsäure umfassen, die das Reportergen umfasst; und/oder
das Reportergen unter der Kontrolle eines Promotor- oder Antwortelements steht, wobei das Antwortelement vorzugsweise ein NFAT-(nuclear factor of activated T cells)-Antwortelement (NFAT-RE) umfasst; und/oder
der Reporter einen biolumineszenten Reporter, wie eine Luciferase, umfasst; und/oder
die Effektor-T-Zellen eine heterologe Nukleinsäure umfassen, die LAG-3 kodiert; und/oder
der T-Zell-Aktivator ein zellfreier T-Zell-Aktivator ist; und/oder
der Anti-CD3-Antikörper OKT3 ist; und/oder
der Anti-CD3-Antikörper oder ein Fragment oder Derivat davon in einer Konzentration vorhanden ist, die seine Verwendung im Assay bei einer Konzentration von ~6-30 x $10^{-12}$ M (1-4 ng/ml für den gesamten Antikörper oder das molare Äquivalent für ein Fragment oder Derivat davon) ermöglicht; und/oder
das Kit ferner ein Molekül des gleichen Typs wie der Agonist umfasst, dem jedoch bekanntlich Agonistenaktivität für LAG-3 fehlt, zur Verwendung als Negativkontrolle; und/oder
das Kit ferner einen bekannten LAG-3-Agonisten zur Verwendung als Positivkontrolle umfasst; und/oder
die Effektor-T-Zellen von Jurkat abgeleitete Zellen umfassen; und/oder
die Effektor-T-Zellen Jurkat-LAG-$3^+$/NFAT-luc2-Zellen umfassen; und/oder
das Kit keine APCs, aAPCs oder andere MHC-Klasse-I- oder MHC-Klasse-11-exprimierenden Zellen umfasst; und/oder
die einzigen Zellen in dem Kit die Effektor-T-Zellen sind.

**14.** Kit nach Anspruch 12 oder 13 zur Bestimmung der Wirkstärke eines Präparats eines LAG-3-Agonisten.

**15.** Kit nach Anspruch 12 oder 13 zur Durchführung eines Assays nach einem der Ansprüche 1 bis 11.

**16.** Verwendung eines Kits nach Anspruch 12 oder 13 zur Bestimmung der Aktivität eines LAG-3-Agonisten, zur Bestimmung der Wirkstärke eines Präparats eines LAG-3-Agonisten oder zum Screening auf einen LAG-3-Agonisten.

## Revendications

**1.** Essai *in vitro* de détermination de l'activité d'un agoniste du gène 3 d'activation des lymphocytes (LAG-3), qui comprend :

fournir une pluralité de cellules T effectrices, chaque cellule T effectrice exprimant LAG-3 et un récepteur des cellules T (TCR) sur sa surface, et comprenant un gène rapporteur codant un rapporteur, dans lequel l'expression du rapporteur est régulée par l'inhibition médiée par LAG-3 de la signalisation de TCR dans les cellules T effectrices ; et
déterminer l'activité de l'agoniste d'après l'étendue selon laquelle l'expression du rapporteur est altérée en présence de l'agoniste comparée à l'expression du rapporteur en l'absence de l'agoniste ;
où l'essai est effectué en l'absence d'un ligand naturel pour LAG-3, dans lequel le ligand naturel est le MHC de classe II.

**2.** Essai selon la revendication 1, de détermination de la puissance d'une préparation d'un agoniste de LAG-3.

**3.** Essai *in vitro* de criblage pour un agoniste de LAG-3, qui comprend :

fournir une pluralité de cellules T effectrices, chaque cellule T effectrice exprimant LAG-3 et un récepteur des cellules T (TCR) sur sa surface, et comprenant un gène rapporteur codant un rapporteur, dans lequel l'expression du rapporteur est régulée par l'inhibition médiée par LAG-3 de la signalisation de TCR dans les cellules T effectrices ; et

déterminer si un agoniste candidat est un agoniste de LAG-3 en déterminant l'étendue selon laquelle l'expression du rapporteur est altérée en présence de l'agoniste candidat comparée à l'expression du rapporteur en l'absence de l'agoniste candidat ;

où l'essai est effectué en l'absence d'un ligand naturel pour LAG-3, dans lequel le ligand naturel est le MHC de classe II.

**4.** Essai selon la revendication 1 ou 2, dans lequel le rapporteur est exprimé à un niveau basal dans les cellules T effectrices en l'absence de l'agoniste, ou un essai selon la revendication 3, dans lequel le rapporteur est exprimé à un niveau basal dans les cellules T effectrices en l'absence de l'agoniste candidat.

**5.** Essai selon l'une quelconque des revendications précédentes, dans lequel l'expression du rapporteur est réduite en présence de l'agoniste ou de l'agoniste candidat, comparée à l'expression du rapporteur en l'absence de l'agoniste ou de l'agoniste candidat.

**6.** Essai selon l'une quelconque des revendications précédentes, dans lequel l'expression du rapporteur est altérée dans chaque cellule T effectrice en réponse à l'activation de la cellule T effectrice via le TCR, et qui comprend en outre :

activer les cellules T effectrices par activation des cellules T effectrices médiée par TCR, indépendante d'un antigène, indépendante du MHC de classe II, en présence et en l'absence de l'agoniste ou de l'agoniste candidat, dans lequel les cellules T effectrices sont activées en mettant les cellules T effectrices en contact avec un activateur de cellules T indépendant d'un antigène, indépendant du MHC de classe II, dans des conditions d'activation des cellules T effectrices médiée par TCR, indépendante d'un antigène, indépendante du MHC de classe II, par un activateur de cellules T, et dans lequel l'activateur de cellules T comprend ou est constitué d'un anticorps anti-CD3, ou d'un fragment ou dérivé du même qui conserve la capacité d'activation des cellules T effectrices médiée par TCR, indépendante d'un antigène, indépendante du MHC de classe II ; et

déterminer l'activité de l'agoniste ou de l'agoniste candidat, d'après l'étendue selon laquelle l'expression du rapporteur est altérée, en réponse à l'activation des cellules T effectrices, en présence de l'agoniste ou de l'agoniste candidat, comparée à l'expression du rapporteur, en réponse à l'activation des cellules T effectrices, en l'absence de l'agoniste ou de l'agoniste candidat ; optionnellement

dans lequel l'expression du rapporteur est augmentée dans chaque cellule T effectrice en réponse à l'activation de la cellule T effectrice via le TCR, et est réduite en présence de l'agoniste ou de l'agoniste candidat, suite à l'inhibition médiée par LAG-3 de la signalisation de TCR dans la cellule T effectrice, et dans lequel l'activité de l'agoniste ou de l'agoniste candidat, est déterminée d'après l'étendue selon laquelle l'expression du rapporteur est réduite, en réponse à l'activation des cellules T effectrices, en présence de l'agoniste ou de l'agoniste candidat, comparée à l'expression du rapporteur, en réponse à l'activation des cellules T effectrices, en l'absence de l'agoniste ou de l'agoniste candidat.

**7.** Essai selon la revendication 6, dans lequel les cellules T effectrices sont mises en contact avec l'activateur de cellules T à une concentration de l'activateur de cellules T à laquelle une inhibition maximale de l'expression du rapporteur survient en présence d'un excédent d'un agoniste de LAG-3.

**8.** Essai selon la revendication 7, dans lequel les cellules T effectrices sont mises en contact avec l'activateur de cellules T à une concentration de l'activateur de cellules T qui est moindre qu'une concentration de l'activateur de cellules T à laquelle l'expression la plus haute du rapporteur est observée en réponse à l'activation des cellules T effectrices par l'activateur de cellules T en l'absence de l'agoniste ou de l'agoniste candidat.

**9.** Essai selon l'une quelconque des revendications 6 à 8, dans lequel l'anticorps anti-CD3 est OKT3, et/ou l'anticorps anti-CD3, ou fragment ou dérivé du même est mis en contact avec les cellules T effectrices à une concentration de ~6-30 x $10^{-12}$ M (1-4 ng/ml pour l'anticorps entier, ou l'équivalent molaire pour un fragment ou dévíré du même).

**10.** Essai selon l'une quelconque des revendications précédentes, dans lequel les cellules T effectrices sont activées par activation des cellules T effectrices, acellulaire, indépendante d'un antigène, indépendante du MHC de classe II, dans lequel les cellules T effectrices sont activées en mettant les cellules T effectrices en contact avec un activateur de cellules T indépendant d'un antigène, indépendant du MHC de classe II, dans des conditions d'activation médiée par TRC des cellules T effectrices indépendante d'un antigène, indépendante du MHC de classe II, par l'activateur de cellules T, et dans lequel l'activateur de cellules T comprend ou est constitué d'un anticorps anti-CD3, ou d'un fragment ou dérivé du même qui conserve la capacité d'activation de cellules T effectrices médiée par TCR indépendante d'un antigène, indépendante du MHC de classe II, et/ou dans lequel les cellules T effectrices sont mises en contact avec plusieurs différentes concentrations de l'agoniste ou de l'agoniste candidat, optionnellement où l'essai comprend en outre déterminer une valeur de $CI_{50}$ de l'agoniste ou de l'agoniste candidat, pour l'inhibition de l'expression du rapporteur.

**11.** Essai selon l'une quelconque des revendications précédentes, dans lequel :

les cellules T effectrices comprennent un acide nucléique hétérologue comprenant le gène rapporteur ; et/ou
le gène rapporteur est sous le contrôle d'un promoteur ou élément de réponse, de préférence dans lequel l'élément de réponse comprend un élément de réponse NFAT (facteur nucléaire des cellules T activées) (NFAT-RE); et/ou
le rapporteur comprend un rapporteur bioluminescent, tel qu'une luciférase ; et/ou
les cellules T effectrices comprennent un acide nucléique hétérologue codant LAG-3 ; et/ou
l'essai comprend en outre faire un essai de contrôle négatif, dans lequel les cellules T effectrices sont activées en l'absence de l'agoniste ou de l'agoniste candidat, mais en présence d'une molécule du même type que l'agoniste ou l'agoniste candidat, mais qui est connue pour être dépourvue d'activité agoniste pour LAG-3 ; et/ou
l'agoniste ou l'agoniste candidat est un anticorps anti-LAG-3, ou un fragment ou dérivé du même qui conserve une activité agoniste anti-LAG-3 ; et/ou
les cellules T effectrices comprennent des cellules dérivées de Jurkat ; et/ou
l'agoniste est un anticorps agoniste anti-LAG3, ou un fragment ou dérivé du même qui conserve une activité agoniste anti-LAG-3, et les cellules T effectrices comprennent des cellules $LAG-3^+$/NFAT-luc2 Jurkat ; et/ou
l'agoniste est un anticorps anti-LAG-3, ou un fragment ou dérivé du même, qui comprend des séquences de CDR1-3 VH et des séquences de CDR1-3 VL des SEQ ID NO : 1-6 ou des SEQ ID NO : 7-12, respectivement ; et/ou
l'agoniste est l'anticorps anti-LAG-3 IMP761.

**12.** Kit pour effectuer un essai *in vitro* de détermination d'activité, ou de criblage, d'un agoniste de LAG-3, qui comprend :

une pluralité de cellules T effectrices, chaque cellule T effectrice exprimant LAG-3 et un récepteur des cellules T (TCR) sur sa surface, et comprenant un gène rapporteur codant un rapporteur, dans lequel l'expression du rapporteur est régulée par l'inhibition médiée par LAG-3 de la signalisation de TCR dans les cellules T effectrices, et dans lequel l'expression du rapporteur est altérée dans chaque cellule T effectrice en réponse à l'activation de la cellule T effectrice via le TCR ; et
un activateur de cellules T capable d'activation médiée par TCR des cellules T effectrices indépendante d'un antigène, indépendante du MHC de classe II, dans lequel l'activateur de cellules T comprend un anticorps anti-CD3, ou un fragment ou dérivé du même qui conserve une capacité d'activation de cellules T effectrices médiée par TCR indépendante d'un antigène, indépendante du MHC de classe II ;
où le kit ne comprend aucun ligand naturel pour LAG-3, où le ligand naturel est le MHC de classe II.

**13.** Kit selon la revendication 12, dans lequel :

le kit ne comprend aucune cellule exprimant des molécules MHC de classe II ;
et/ou
l'expression du rapporteur est augmentée en réponse à l'activation des cellules T effectrices via le TCR ; et/ou
les cellules T effectrices comprennent un acide nucléique hétérologue comprenant le gène rapporteur ; et/ou
le gène rapporteur est sous le contrôle d'un promoteur ou élément de réponse, de préférence dans lequel l'élément de réponse est un élément de réponse NFAT (facteur nucléaire de cellules T activées) (NFAT-RE) ; et/ou
le rapporteur comprend un rapporteur bioluminescent, tel que la luciférase ; et/ou
les cellules T effectrices comprennent un acide nucléique hétérologue codant LAG-3 ; et/ou
l'activateur de cellules T est un activateur de cellules T acellulaire ; et/ou
l'anticorps anti-CD3 est OKT3 ; et/ou
l'anticorps anti-CD3, ou fragment ou dérivé du même, est présent à une concentration qui en permet l'utilisation dans l'essai à une concentration de ~6-30 x $10^{-12}$ M (1-4 ng/ml pour un anticorps entier, ou l'équivalent molaire pour un fragment ou dérivé du même) ; et/ou
le kit comprend en outre une molécule du même type que l'agoniste, mais qui est connue pour être dépourvue d'activité agoniste pour LAG-3, pour utilisation comme un témoin négatif ; et/ou
le kit comprend en outre un agoniste connu de LAG-3 pour utilisation comme un témoin positif ; et/ou

les cellules T effectrices comprennent des cellules dérivées de Jurkat ; et/ou
les cellules T effectrices comprennent des cellules LAG-$3^+$/NFAT-luc2 Jurkat ; et/ou
le kit ne comprend aucune cellule APC, aAPC, ou n'importe quelles autres cellules exprimant le MHC de classe I ou le MHC de classe II ; et/ou
les seules cellules dans le kit sont les cellules T effectrices.

**14.** Kit selon la revendication 12 ou 13, pour déterminer la puissance d'une préparation d'un agoniste de LAG-3.

**15.** Kit selon la revendication 12 ou 13 pour faire un essai selon l'une quelconque des revendications 1 à 11.

**16.** Utilisation d'un kit selon la revendication 12 ou 13 pour déterminer l'activité d'un agoniste de LAG-3, déterminer la puissance d'une préparation d'un agoniste de LAG-3, ou cribler pour un agoniste de LAG-3.

Figure 1

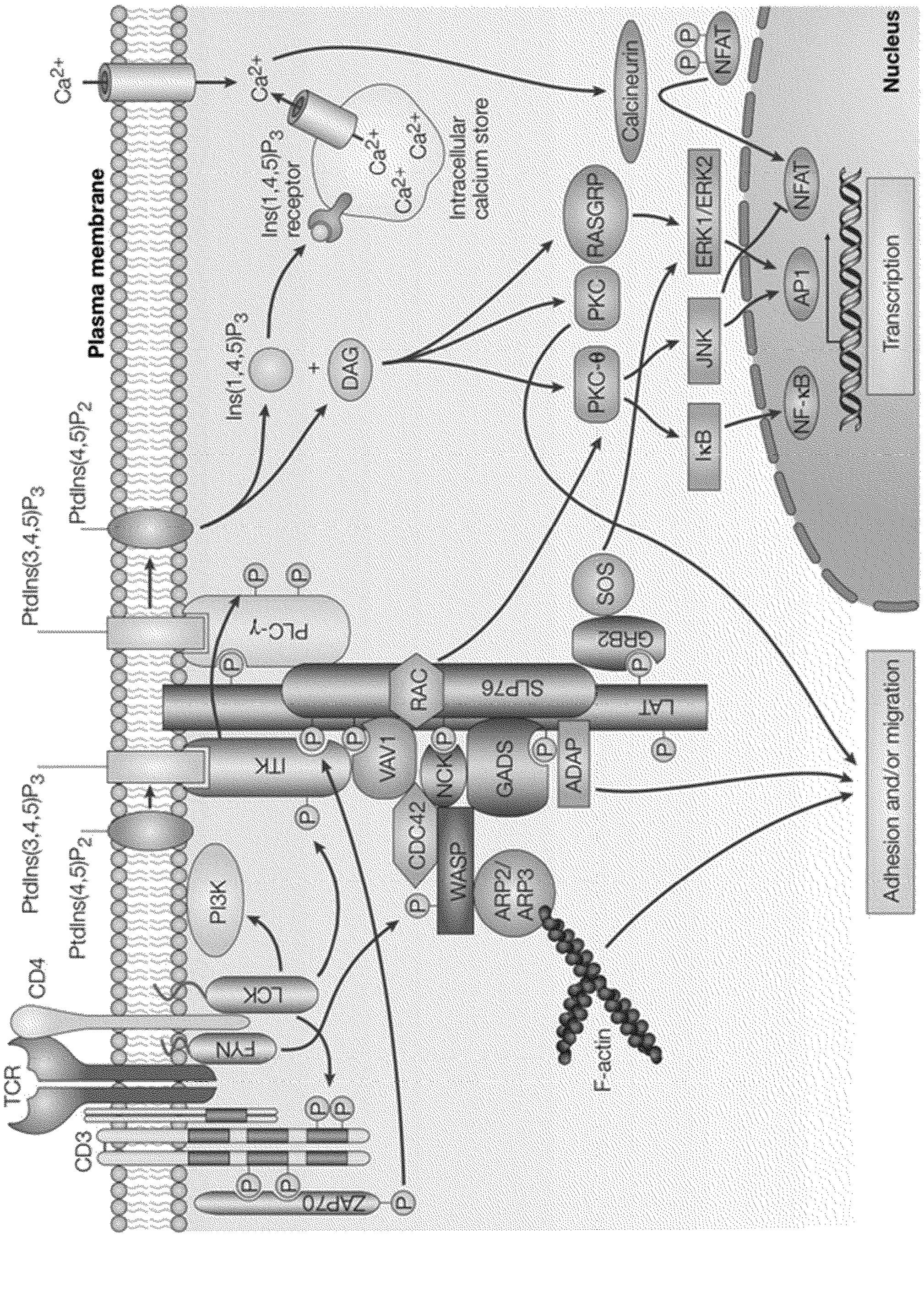
TCR
CD3
CD4
ZAP70
FYN
LCK
PI3K
P
PtdIns(4,5)$P_2$
PtdIns(3,4,5)$P_3$
Plasma membrane
ITK
PLC-γ
VAV1
CDC42
NCK
WASP
ARP2/ ARP3
F-actin
GADS
ADAP
LAT
SLP76
RAC
GRB2
SOS
Ins(1,4,5)$P_3$
DAG
Ins(1,4,5)$P_3$ receptor
$Ca^{2+}$
Intracellular calcium store
Calcineurin
PKC-θ
PKC
RASGRP
IκB
JNK
ERK1/ERK2
NF-κB
AP1
NFAT
Transcription
Nucleus
Adhesion and/or migration

Figure 2

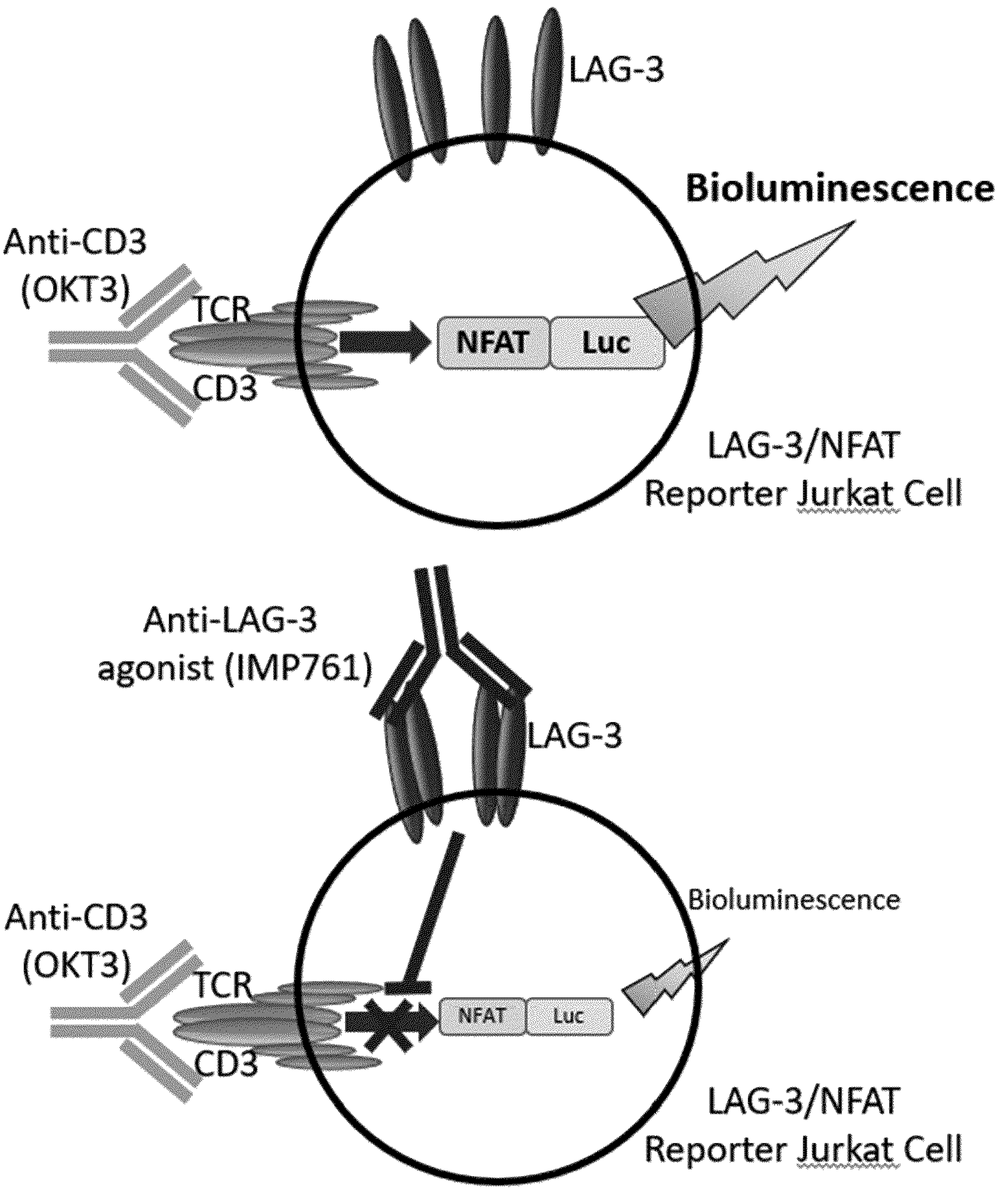
LAG-3
Bioluminescence
Anti-CD3
(OKT3)
TCR
CD3
NFAT
Luc
LAG-3/NFAT
Reporter Jurkat Cell
Anti-LAG-3
agonist (IMP761)
LAG-3
Bioluminescence
Anti-CD3
(OKT3)
TCR
CD3
NFAT
Luc
LAG-3/NFAT
Reporter Jurkat Cell

Figure 3

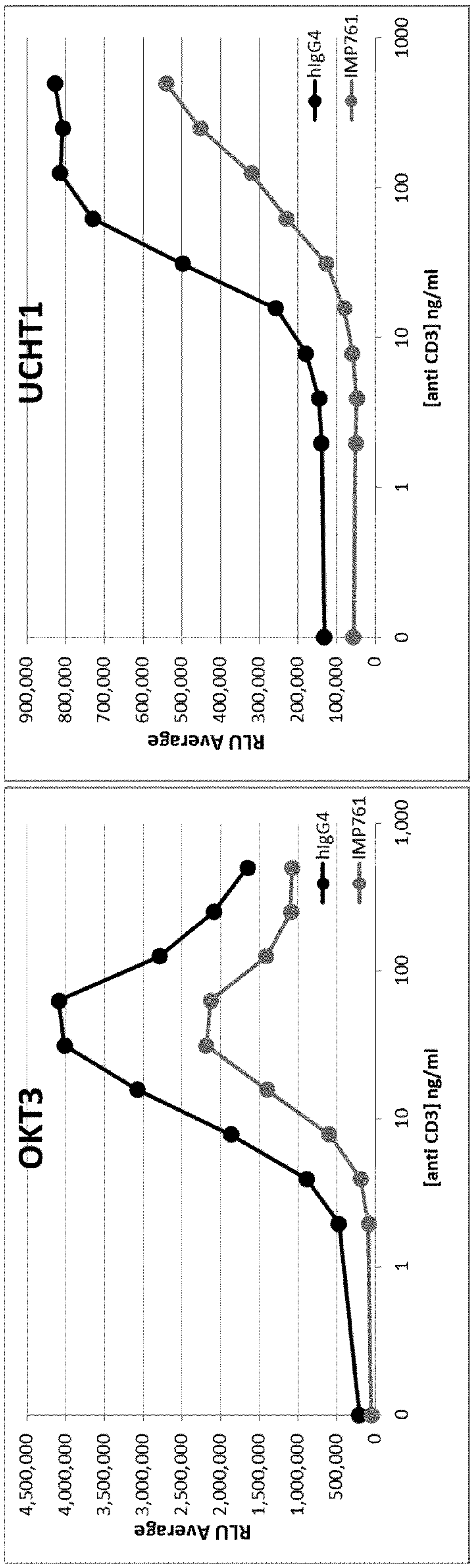
OKT3
RLU Average
4,500,000
4,000,000
3,500,000
3,000,000
2,500,000
2,000,000
1,500,000
1,000,000
500,000
0
0
1
10
100
1,000
[anti CD3] ng/ml
hIgG4
IMP761
UCHT1
RLU Average
900,000
800,000
700,000
600,000
500,000
400,000
300,000
200,000
100,000
0
0
1
10
100
1000
[anti CD3] ng/ml
hIgG4
IMP761

Figure 4

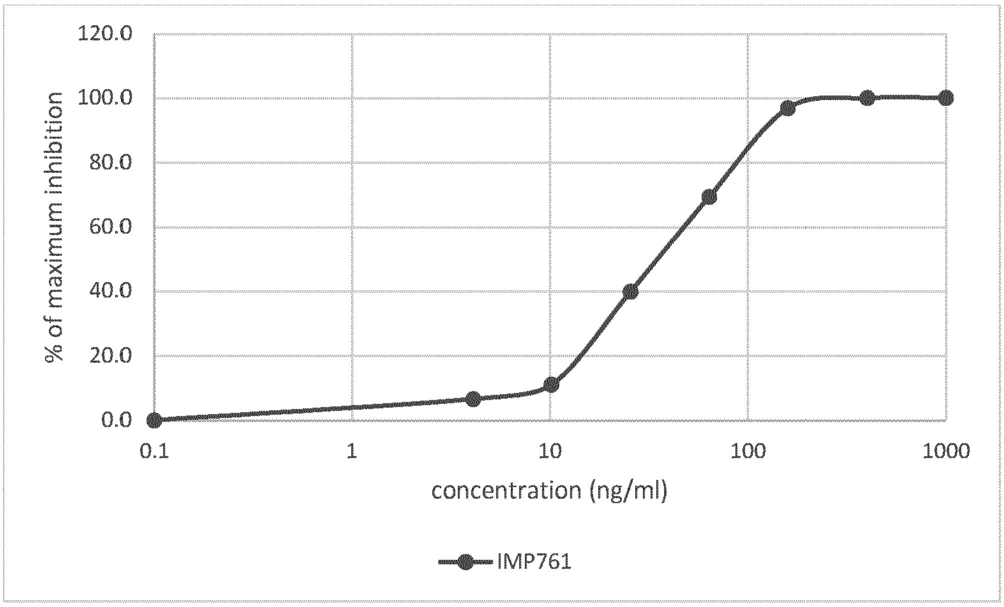
120.0
100.0
80.0
60.0
40.0
20.0
0.0
% of maximum inhibition
0.1
1
10
100
1000
concentration (ng/ml)
IMP761

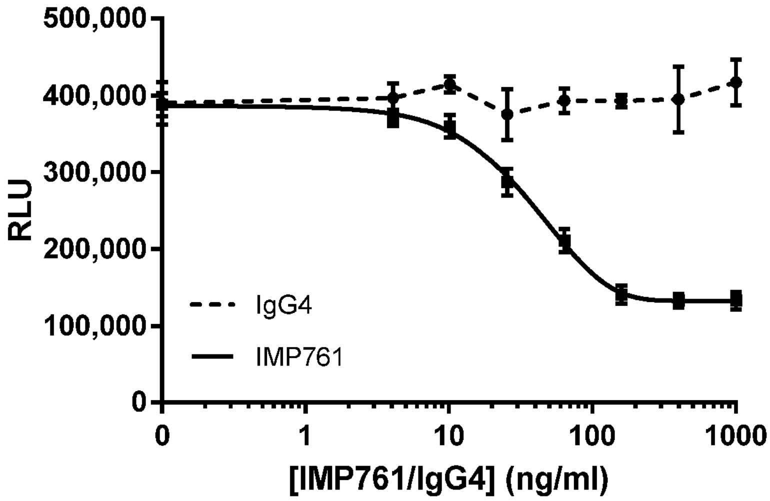
500,000
400,000
300,000
200,000
100,000
0
RLU
IgG4
IMP761
0
1
10
100
1000
[IMP761/IgG4] (ng/ml)

Figure 5

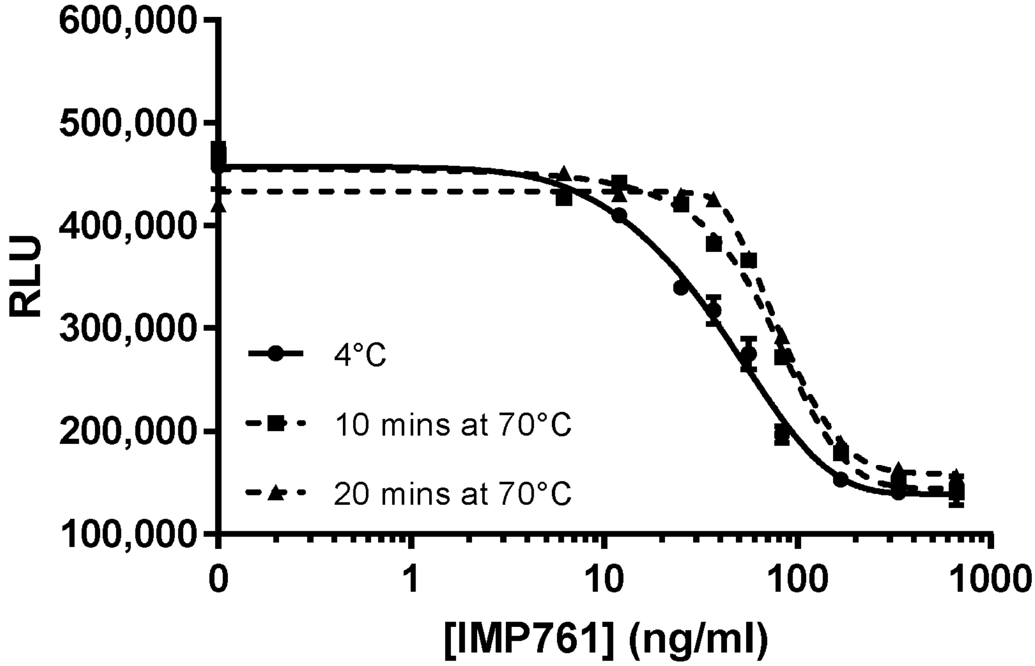
600,000
500,000
400,000
300,000
200,000
100,000
RLU
0
1
10
100
1000
[IMP761] (ng/ml)
4°C
10 mins at 70°C
20 mins at 70°C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017037203 A
- US 2017097333 A1


### Non-patent literature cited in the description


- **MAÇON-LEMAÎTRE et al.** *Immunology*, June 2005, vol. 115 (2), 170-178
- **MAEDA et al.** *J. Biol. Chem.*, 2019
- **CHENG et al.** Quantitative Cell-Based Bioassays for Indivisual and Combination Immune Checkpoint Immunotherapy Targets. *Promega*, June 2016
- **FAN ; WOOD**. Bioluminescent assays for high-throughput screening. *Assay Drug Dev. Technol.*, 2007, vol. 5, 127-36
- **SCHNEIDER et al.** *Int J Cancer*, 1977, vol. 19 (5), 621-6
- **BELIKOV ; ALEKSEY**. *The role of reactive oxygen species and mitochondria in T-cell activation*, 2016